(19) Europäisches Patentamt
European Patent Office
Office européen des brevets

(11) Veröffentlichungsnummer: **0 394 908**
**A1**

# (12) EUROPÄISCHE PATENTANMELDUNG

(21) Anmeldenummer: **90107638.0**

(22) Anmeldetag: **23.04.90**

(51) Int. Cl.5: **C07H 17/04, C07H 15/12, //A61K31/70**

(30) Priorität: **22.04.89 DE 3913327**

(43) Veröffentlichungstag der Anmeldung:
**31.10.90 Patentblatt 90/44**

(84) Benannte Vertragsstaaten:
**AT BE CH DE DK ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Kolar, Cenek, Dr.**
**Deutschhausstrasse 20**
**D-3550 Marburg(DE)**
Erfinder: **Dehmel, Konrad**
**Blumengarten 11**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

## (54) Verfahren zur Herstellung von Glucosaminyl-epi-podo-phyllotoxin-Derivaten.

(57) Die Erfindung betrifft ein neues Verfahren zur Herstellung von Glucosaminyl-epi-podophyllotoxin-Derivaten der Formel I

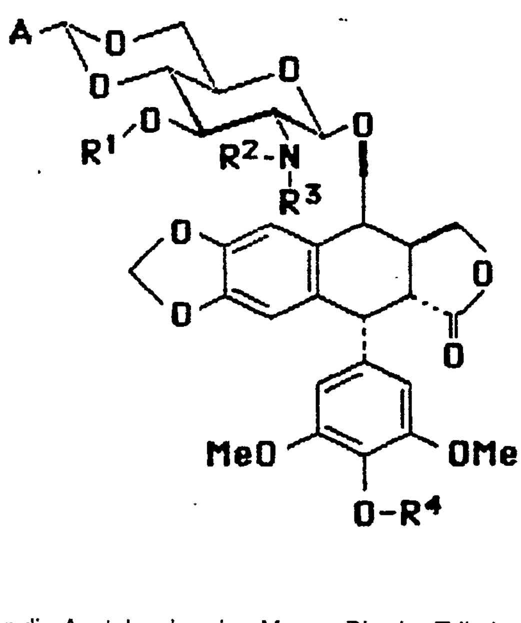

I

worin
$R^1$ Wasserstoff oder die Acetyl- oder eine Mono-, Di-oder Trihalogenacetyl-Schutzgruppe mit Halogen Fluor, Chlor oder Brom,
$R^2$ Wasserstoff oder $(C_1-C_4)$-Alkyl,
$R^3$ Wasserstoff, $(C_1-C_4)$-alkyl oder eine Benzyloxycarbonyl-Schutzgruppe,
$R^4$ Wasserstoff, eine Mono-, Di- oder Trihalogenacetyl-Schutzgruppe, eine Benzyloxycarbonyl-Schutzgruppe oder eine Methylgruppe und

EP 0 394 908 A1

A $C_1$-$C_4$-Alkyl
bedeuten, dadurch gekennzeichnet, daß man ein in der alpha-Hydroxy- Form vorliegendes 3-O-Acyl-2-N-Benzyloxycarbonyl-4,6-O-alkyliden-alpha-D-glucosamin-Derivat der Formel II

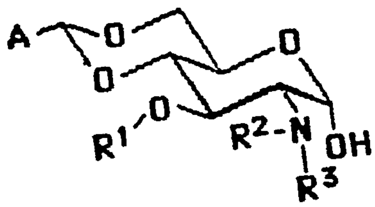

II

worin
$R^1$ und $R^3$ wie oben definiert Acyl-Schutzgruppen sind und
$R^2$ Wasserstoff ist,
mit einem Podophyllotoxin-Derivat der Formel III

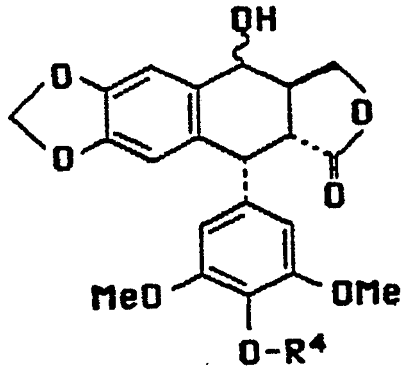

III

worin
$R^4$ die Methylgruppe oder eine Mono-, Di- oder Trihalogenacetyl- oder Benzyloxycarbonyl-Schutzgruppe darstellt, in der Gegenwart eines Promotors und eines wasserfreien organischen Lösungsmittels bei -50° C bis 20° C zu 4-O-(beta-Glucosaminyl)-epi-Podophyllotoxin-Derivat der Formel I, in der die Reste $R^1$, $R^2$, $R^3$ und $R^4$ ihre Bedeutung, wie oben definiert, beibehalten, umsetzt und anschließend die Glycosid-Produkte der Formel I nach Abspaltung der Schutzgruppen in eine Amino- oder Dialkylamino-Verbindung der Formel I überführt.

## Verfahren zur Herstellung von Glucosaminyl-epi-podophyllotoxin-Derivaten

Die vorliegende Erfindung bezieht sich auf ein Verfahren zur Herstellung von Glucosaminyl-epi-podophyllotoxin-Derivaten, besonders 4-O-(4,6-O-Alkyliden-beta-D-glucosaminyl)-4′-demethyl-4-epi-podophyllotoxin-Derivaten, die aufgrund ihrer zytostatischen Wirksamkeit für die Behandlung von Krebserkrankungen geeignet sind.

Glycosyl-epipodophyllotoxine sind in der Fachliteratur beschrieben. Zwei Vertreter dieser Substanzklasse, Etoposid und Teniposid, sind als Arzneimittel zur Behandlung von Krebserkrankungen eingeführt. Etoposid-Derivate, die statt der Glucoseeinheit einen Glucosamin-oder N,N-Dimethyl-glucosamin-Kohlenhydratbaustein enthalten, zeigen bei der präklinischen Untersuchung ein breiteres Wirksamkeitsspektrum als Etoposid. Da diese Verbindungen aufgrund der Präsenz der Aminogruppe gut in wäßrigem Medium löslich sind, ist die Herstellung galenischer Darreichungsformen von ihnen verglichen mit Etoposid unproblematisch.

In EP-A-0 141 057 und 0 196 618 sind Etoposid-Derivate beschrieben, die statt der Glucose einen Glucosamin-oder N,N-Dimethyl-glucosamin-Baustein enthalten.

Aus der genannten Anmeldung EP-A-0 141 057 ist auch ein Verfahren bekannt, bei dem die Darstellung der erfindungsgemäßen Verbindungen durch Umsetzung von einem funktionalisierten beta-Glucosamin-Baustein und 4′-O-geschütztem epi-Podophyllotoxin unter $BF_3$-Katalyse erfolgt. Dieses Verfahren ist jedoch aufwendig und verlustreich, da man ausgehend von einer nur aufwendig herstellbaren und aufgrund der Mutarotation instabilen beta-Hydroxy-Glycosylierungskomponente das gewünschte epi-Podophyllotoxin-Glycosid herstellt. Auch die folgende Entfernung der Acyl-Schutzgruppen sowie die Einführung der 4″,6″-Alkylidengruppe ist wegen der Vielzahl der Nebenprodukte verlustreich und erfordert zusätzliche chromatographische Reinigungsschritte.

Aus Chemistry Letters, 799-802, 1987 ist ein Verfahren zur Synthese der beta-Glucosaminyl-epi-podophyllotoxine bekannt, in dem die Glycosylierungskomponente als 4,6-O-Ethylidenglucosamin-Derivat eingesetzt wird. Dies funktionalisierte Glucosamin-Komponente, die in der instabilen beta-Hydroxy-Form vorliegt, ergibt nach der Glycosylierung des Aglycons und anschließender Abspaltung der Schutzgruppen das gewünschte beta-Glu- cosaminyl-epi-podophyllotoxin in 33%iger Ausbeute.

Es wurde überraschenderweise festgestellt, daß bei der Glycosylierung von 4′-O-Z-4-epi-podophyllotoxin (Z: benzyloxycarbonyl) mit einem funktionalisierten, in der alpha-Hydroxy-Form vorliegenden Glucosamin-Baustein unter $BF_3$-Katalyse in Essigsäureethylester bevorzugt beta-Glycosid entsteht. Die beta-Glycosylierung von epi-Podophyllotoxinen wie bisher in der Fachliteratur beschrieben, verläuft über das Carboniumion am C-4-Atom des Aglycons, wobei der nucleophile Angriff der beta-Hydroxygruppe des Glucosamin-Bausteines erforderlich ist. Da die alpha-Hydroxy-Form der Glycosylierungseinheit aufgrund des anomeren Effektes energetisch stabiler ist als die beta-Hydroxy-Form, wobei die alpha-Hydroxy-Glucosamin-Derivate synthetisch einfacher als die beta-Hydroxy-Analoga zugänglich sind, stellt das erfindungsgemäße Verfahren einen neuen vorteilhaften Weg zur technischen Herstellung der beta-Glucosaminyl-epi-podophyllotoxine dar.

Der Erfindung liegt die Aufgabe zugrunde, ein neues Verfahren zu entwickeln, das 4-O-(4,6-O-Alkyliden-beta-D-glucosaminyl)-4′-O-demethyl-4-epi-podophyllotoxin-Derivate in guten Ausbeuten liefert und das eine Vereinfachung gegenüber den bekannten Verfahren bedeutet. Insbesondere sollen ein neues Verfahren zur Herstellung einer geeigneten Glucosamin-Glycosidierungkomponente sowie neue Deacylierungsverfahren zur Entfernung der Acyl-Schutzgruppe von Glucosaminyl-epi-podophyllotoxinen entwickelt werden.

Diese Aufgabe wird erfindungsgemäß gelöst durch das Verfahren zur Herstellung eines Glucosaminyl-epi-podophyllotoxin-Derivates der Formel I

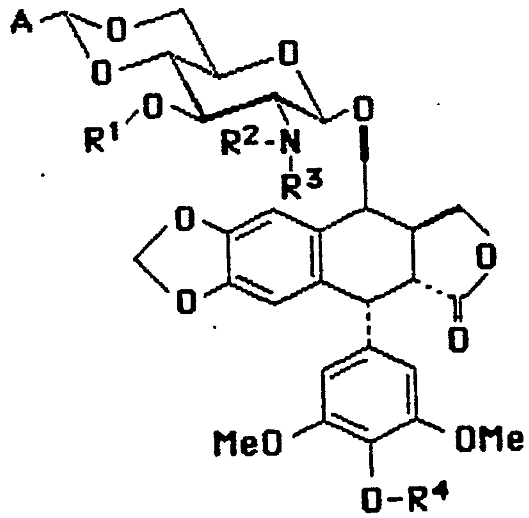

I

worin
$R^1$ Wasserstoff oder die Acetyl- oder eine Mono-, Di-oder Trihalogenacetyl-Schutzgruppe mit Halogen Fluor, Chlor oder Brom,
$R^2$ Wasserstoff oder $(C_1\text{-}C_4)$-Alkyl,
$R^3$ Wasserstoff, $(C_1\text{-}C_4)$-Alkyl oder eine Benzyloxycarbonyl-Schutzgruppe,
$R^4$ Wasserstoff, eine Mono-, Di- oder Trihalogenacetyl-Schutzgruppe, eine Benzyloxycarbonyl-Schutzgruppe oder eine Methylgruppe und
A $C_1\text{-}C_4$-Alkyl
bedeuten, dadurch gekennzeichnet, daß man ein in der alpha-Hydroxy-Form vorliegende Glucosamin-Derivat der Formel II

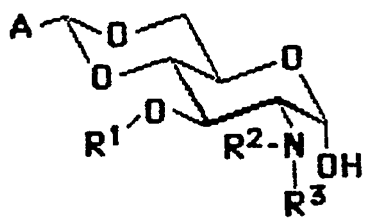

II

worin
$R^1$ und $R^3$ wie oben definiert Acyl-Schutzgruppen sind und
$R^2$ Wasserstoff und
A $C_1\text{-}C_4$-Alkyl bedeuten,
mit einem Podophyllotoxin-Derivat der Formel III

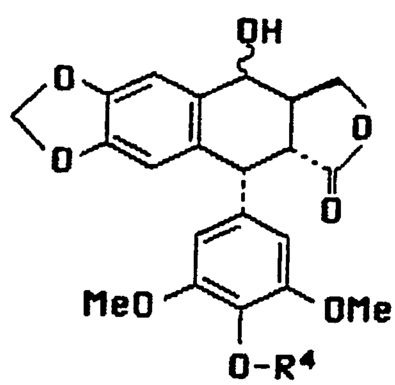

III

worin
$R^4$ eine Methylgruppe oder eine Mono-, Di- oder Trihalogenacetyl- oder Benzyloxycarbonyl-Schutzgruppe darstellt,
in der Gegenwart eines Promotors wie $Bf_3$xEther oder eines Tri-($C_1$-$C_4$)-alkyl-silyltrifluormethansulfonates und eines wasserfreien organischen Lösungsmittels bei -50° C bis 20° C zu einem 4-O-(beta-Glucosaminyl)-epi-Podophyllotoxin-Derivat der Formel I, in der die Reste $R^1$, $R^2$, $R^3$, $R^4$ und A ihre Bedeutung wie oben definiert, beibehalten, anschließend die Benzyloxycarbonyl-Schutzgruppe hydrogenolytisch mittels eines Palladium-Katalysators, die Acetylschutzgruppe mittels Zink-dichlorid oder -diacetat und die Halogenacetylschutzgruppen mittels eines basischen Anionenaustauschers in einem organischen Lösungsmittel, wie Methanol, Ethanol, Aceton, Essigsäureethylester oder deren gegebenenfalls wäßrigen Mischungen abspaltet und gegebenenfalls die freie Aminogruppe unter den Bedingungen der reduktiven Alkylierung mit einem $C_1$-$C_4$-Aldehyd in der Gegenwart eines Hydrides zu einem Mono- oder Dialkylamino-Derivate umsetzt, wobei ein 4-O-(beta-Glucosaminyl)-epi-podophyllotoxin-Derivat der Formel I, worin $R^1$ Wasserstoff,
$R^2$ und $R^3$ Wasserstoff oder ($C_1$-$C_4$)-Alkyl,
$R^4$ Wasserstoff oder die Methylgruppe und
A $C_1$-$C_4$-Alkyl
darstellen, gebildet wird.

Bevorzugt werden nach dem erfindungsgemäßen Verfahren Verbindungen der Formel I hergestellt, worin
$R^1$ Wasserstoff oder Acetyl- oder Chloracetyl-Schutzgruppe
$R^2$ Wasserstoff oder ($C_1$-$C_4$)-Alkyl
$R^3$ Wasserstoff, ($C_1$-$C_4$)-Alkyl oder Benzyloxycarbonyl-Schutzgruppe
$R^4$ Wasserstoff, Methyl oder Benzyloxycarbonyl- oder Chloracetyl-Schutzgruppen und
A Methyl bedeuten.

Im einzelnen geht man dabei wie folgt vor: Zunächst wird, ausgehend von dem 1,3-Di-O-acyl-Vorprodukt der Formel IV

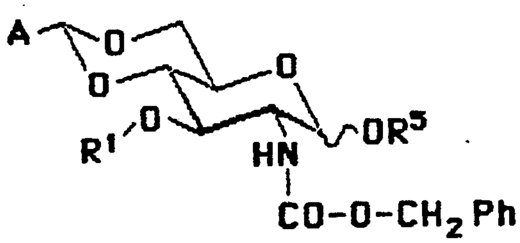

IV

die Glucosamin-Glycosidierungskomponente der Formel V

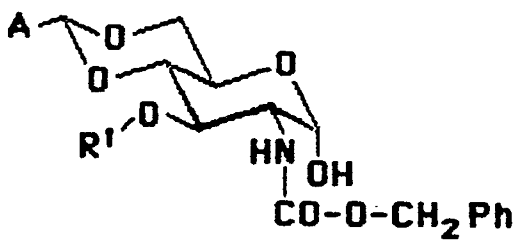

V

worin $R^1$ und $R^5$ eine Acetyl- oder Mono-, Di- oder Trihalogenacetyl-Schutzgruppe und A $C_1$-$C_4$-Alkyl darstellen, auf folgende Weise hergestellt: wenn $R^5$ eine Acetylgruppe darstellt, wird das Vorprodukt mit einer schwachen organischen Base wie Piperidin oder Pyridin, und wenn $R^5$ eine Halogenacetyl-Schutzgruppe darstellt, wird das Vorprodukt mit Kieselgel in einem polaren organischen Lösungsmittel wie Methanol oder Ethanol behandelt.

Durch Auflösen der Produkte in einem unpolaren Lösungsmittel wie Chloroform, Dichlormethan wird die Glucosamin-Verbindung in die energetisch stabilere alpha-Form überführt.

Die Glycosylierung eines Podophyllotoxin-Derivates der Formel III mit dem Glucosamin-Baustein der Formel V verläuft bevorzugt in Essigsäureethylester, gegebenenfalls unter Beimischung von Dichlormethan, Chloroform, Ether oder Aceton, in der Gegenwart von 1 bis 50 Äquivalenten, bevorzugt 20 bis 30

Äquivalenten $BF_3$-Ether oder Tri-($C_1$-$C_4$)-alkylsilyltrifluormethansulfonat bei -50° C bis 20° C, bevorzugt bei -30° C bis -15° C. Als Säurefänger oder Trockenmittel kann gegebenenfalls ein Molekularsieb verwendet werden.

Die Abspaltung der Schutzgruppe aus dem 4-O-Glucosaminyl-epi-podophyllotoxin-Derivat der Formel I wird wie folgt durchgeführt:

Die Benzyloxycarbonyl-Schutzgruppe wird hydrogenolytisch mittels Palladium/Kohle oder Palladium/Bariumsulfat in Methanol, Ethanol, Aceton oder Essigsäureethylester abgespalten.

Die Acetylschutzgruppe wird mittels wasserfreiem Zinkdiacetat oder -dichlorid in Methanol oder Ethanol unter Rückflußtemperatur abgespalten.

Die Halogenacetyl-Schutzgruppen, bevorzugt die Chloracetyl-Schutzgruppe, werden mittels eines basischen Anionenaustauschers wie Dowex, bevorzugt 1x8 in einem organischen Lösungsmittel wie Methanol, Ethanol, Aceton, Essisäureethylester, bevorzugt Methanol, oder deren Mischungen abgespalten. Die Produkte der Formel I, die eine freie Aminogruppe am C-2″-Atom besitzen, werden in Mono-, bevorzugt jedoch zu Di-($C_1$-$C_4$)-alkylamin-Derivaten mittels der reduktiven Alkylierung mit einem $C_1$-$C_4$-Aldehyd und einem Hydrid wie Natriumcyanoborhydrid überführt.

Die nachfolgenden Beispiele sollen die Erfindung erläutern, jedoch ohne sie auf die genannten Verbindungen zu beschränken.

## Beispiel 1

Herstellung von 2-N-Benzyloxycarbonyl-1,3-di-O-acyl-4,6-O-ethyliden-alpha,beta-D-glucosamin-Derivaten

2-N-Benzyloxycarbonyl-4,6-O-ethyliden-D-glucosamin (Verbindung 1)

Die Titelverbindung wurde nach dem Verfahren von H. Saito et al., wie in Chemistry Letters, 799-802 (1987) beschrieben, wie folgt hergestellt: Ausgehend von D-Glucosamin-Hydrochlorid und Benzyloxycarbonylchlorid wurde in der Gegenwart von 2 Äquivalenten NaOH in Wasser 2-N-Benzyl-oxycarbonyl-D-glucosamin hergestellt, der anschließend mit Acetaldehyd unter $H_2SO_4$-Katalyse zu der Titelverbindung überführt wurde.

1,3-Di-O-acetyl-2-N-benzyloxycarbonyl-4,6-O-ethyliden-alpha,beta-D-glucosamin (Verbindung 2)

Die Verbindung 1 wurde nach dem in der Kohlehydratchemie üblichen Acylierungsverfahren mittels Acetanhydrid und Pyridin zu der Titelverbindung umgesetzt.

2-N-Benzyloxycarbonyl-1,3-bis-O-chloracetyl-4,6-O-ethyliden-alpha, beta-D-glucosamin (Verbindung 3)

33 g (97.2 mmol) Verbindung 1 wurden in 1000 ml Dichlormethan und 300 ml Pyridin gelöst und unter Rühren bei 0° C mit 2.4 Äquivalenten Chloracetylchlorid, gelöst in 500 ml Dichlormethan, portionsweise versetzt. Nach 14 h wurde der Reaktionsansatz mit Natriumphosphat-Puffer, pH 7.5, ausgewaschen, die organische Phase wurde getrocknet und in Vakuum eingedampft. Der Rückstand wurde säulenchromatographisch über Kieselgel (Elutionsmittel: Dichlormethan/Aceton, 10:1) gereinigt.
Ausbeute: 45.4 g (95 %)
1H-NMR: (300 MHz, H,H-COSY, $CDCl_3$) Delta: 7.4-7.3(m, 5H, Ph), 6.20 (d, 1H, J(1,2) = 3.8 Hz, H-1), 5.28 (dd, 1H, J(2,3) = 10.4 Hz, J(3,4) = 9.6 Hz, H-3), 5.11 (d, 1H, Jg = 11.5 Hz, CHPh), 5.03 (d, 1H, Jg, CHPh), 5.02 (d, 1H, J(2,NH) = 9.5 Hz, NH), 4.70 (q, 1H, J(CH,Me) = 5 Hz, CH=), 4.23 (ddd, 1H, J((1,2), J(2,3), J-(2,NH), H-2), 4.15 (s, 2H, $ClCH_2$), 4.13 (dd, 1H, Jg = 10.5 Hz, J(5,6e) = 4.5 Hz, H-6e), 4.02 (d, 1H, Jg = 15 Hz, $ClCH_2$-A), 3.92 (d, 1H, Jg, $ClCH_2$-B), 3.79 (ddd, 1H, J(5,6a) = 10 Hz, J(5.6e), J (4,5), H-5), 3.58 (dd, 1H, J(3,4), J(4,5), H-4), 3.52 (dd, 1H, Jg, J(5,6a), H-6a), 1.33 (d, 3H, J(Me,CH), MeC=)

## Beispiel 2

Herstellung von 3-O-Acyl-2-N-benzyloxycarbonyl-4,6-O-ethyliden-alpha-D-glucosamin-Derivaten

3-O-Acetyl-2-N-benzyloxycarbonyl-4,6-O-ethyliden-alpha-D-glucosamin (Verbindung 4)

2.11 g (5 mmol) Verbindung 2 wurden in 40 ml THF gelöst und mit 0.5 ml Piperidin versetzt. Nach 12 h Rühren wurde die Reaktionsmischung in Vakuum eingedampft und mit Toluol nachdestillert. Der in Chloroform gelöste Rückstand wurde mit Eiswasser ausgewaschen, und die organische Phase wurde über Natriumsulfat getrocknet.

Das Produkt wurde säulenchromatographisch über Kieselgel (Eluenz: Dichlormethan/Aceton, 6:1) gereinigt. Ausbeute: 1.67 g (88 %) Sirup,
$(alpha)_D^{23}$ = +29.1° (c=1 in Chloroform)

2-N-Benzyloxycarbonyl-3-O-chloracetyl-4,6-O-ethyliden-alpha-D-glucosamin (Verbindung 5)

37 g (75.2 mmol) Verbindung 3 wurden in 500 ml Methanol gelöst und mit 74 g Kieselgel 60 (40 - 63 µm) versetzt. Die Suspension wurde 3 h bei RT gerührt. DC: Dichlormethan/Essigsäureethylester, 1:1. Das Reaktionsgemisch wurde abfiltriert und die Festphase mit Methanol nachgewaschen. Nach Eindampfen der organischen Phase wurde der Rückstand in Dichlormethan/Essigsäureethylester gelöst und über 50 g Kieselgel filtiert. Die organische Phase wurde in Vakuum eingedampft und mit Chloroform nachdestilliert. Ausbeute: 28.31 g (91 %) Sirup, $(alpha)_D^{25}$ = +32.9 ° (c=1 in Essigsäureethylester)
1H-NMR (300 MHz, H,H-COSY, $CDCL_3$) Delta: 7.38-7.30 (m, 5H, Ph), 5.32 (dd, 1H, J(2,3) = 11 Hz, J(3,4) = 10 Hz, H-3), 5.26 (d, 1H, J(2,NH) = 10 Hz, NH), 5.23 (d, 1H, J(1,2) = 3.5 Hz, H-1), 5.11 (d, 1H, Jg = 12 Hz, CHPh), 5.03 (d, 1H, Jg = 12 Hz, CHPh), 4.69 (q, 1H, J(Me,CH) = 5 Hz, CH=), 4.09 (dd, 1H, J(5,6e) = 4.7 Hz, Jg = 10 Hz, H-6), 4.04 (d, 1H, J(1,2), J(2,NH), J(2,3), H-2), 4.02 (d, 1H, Jg = 14.6 Hz, ClCH), 3.99 (ddd, 1H, J(4,5) = 10 Hz, J (5,6a) = 10 Hz, J(5,6e) = 4.7 Hz, H-5), 3.92 (d, 1H, Jg, ClCH), 3.52 (dd, 1H, J-(5,6), Jg, H-6a), 3.48 (dd, 1H, J(3,4), J(4,5), H-4), 1.32 (d, 3H, J(Me,CH), MeC=).

## Beispiel 3

Glycosylierung von Podophyllotoxin-Derivaten

4-O-(3-O-Acetyl-2-N-benzyloxycarbonyl-4,6-O-ethyliden-beta-D-glucosaminyl)-4′-O-benzyloxycarbonyl-4′-O-demethyl-4-epi-podophyllotoxin (Verbindung 6)

2.8 g (5.2 mmol) 4′-O-Benzyloxycarbonyl-4′-O-demethyl-4-epi-podophyllotoxin und 1.9 g (5.2 mmol) Verbindung 4 wurde in 300 ml Essigsäureethylester gelöst. Nach der Zugabe von 5 g Molekularsieb 4 Å wurde zu der auf -18° C abgekühlten Reaktionsmischung 39 g $BF_3$xEther zugegeben. Der Reaktionsansatz wurde 6 h bei -18° C gerührt (DC: Dichlormethan/Aceton, 10:1). Dann wurde mit 40 ml Triethylamin neutralisiert und abfiltriert. Die organische Phase wurde in Vakuum eingedampft und der in Chloroform gelöste Rückstand wurde mit Wasser ausgewaschen. Die organische Phase wurde über Natriumsulfat getrocknet und eingedampft. Das resultierende Produkt wurde säulenchromatographisch über Kieselgel (Eluenz: Dichlormethan/Aceton, 10:1) gereinigt.
Ausbeute: 3.6 g( 78 %)
1H-NMR (300 MHZ, H,H-COSY, $CDCl_3$) Delta: 7.38-7.19 (m, 10H, Ph), 6.68 (s, 1H, H-5), 6.44 (s, 1H, H-8), 6.18 (s, 2H, H-2′ und H-6′), 5.88 (s, 1H, H-15a), 5.73 (s, 1H, H-15b), 5.20 (s, 2H, $CH_2$Ph), 5.12 (dd, 1H, J-(2″,3″) = 10 Hz, H-3″), 5.00 (d, 1H, Jg = 12.5 Hz, $CH_2$Ph-A), 4.84 (d, 1H, Jg, $CH_2$Ph-B), 4.82 (d, 1H, J(3,4) = 3 Hz, H-4), 4.79 (d, 1H, J(2″,NH) = 10 Hz, NH), 4.69 (d, 1H, J(1″,2″) = 8.5 Hz, H-1″), 4.69 (q, 1H, J-(CH,Me) = 5 Hz, MeCH=), 4.45 (d, 1H, J(1,2) = 5 Hz, H-1), 4.41 (dd, 1H, J(3,11a) = 9 Hz, Jg = 9 Hz, H-11a), 4.18 (dd, 1H, Jg, J(3,11b) = 7 Hz, H-11b), 4.15 (dd, 1H, Jg = 10 Hz, J(5″,6″e) = 4.5 Hz, H-6″e), 3.60 (s, 3H, MeO), 3.56 (ddd,, 1H, J(1″,2″), J(2″, NH), J(2″,3″), H-2″), 3.54 (dd, 1H, Jg, J(5″,6″a) = 10 Hz, H-6″a), 3.40 (dd, 1H, J(3″,4″), J(4″, 5″) = 9.5 Hz, H-4″), 3.33 (ddd, 1H, J(4″,5″), J(5″,6″a), J(5″,6″e), H-5″), 3.19 (dd, 1H, J(1,2), J(2,3) = 14 Hz, H-2), 2.77 (m, 1H, J(2,3), J(3,4), J(3,11a), J(3,11b), H-3), 1.97 (s, 3H, Acetyl), 1.28 (d, 3H, J(CH,Me) = 5 Hz, MeC=).

4′-O-Benzyloxycarbonyl-4-O-(2-N-benzyloxycarbonyl-3-O-chloracetyl-4,6-O-ethyliden-beta-D-glucosaminyl)-4′-O-demethyl-4-epi-podophyllotoxin (Verbindung 7)

13 g (25 mmol) 4′-O-Benzyloxycarbonyl-4′-O-demethyl-4-epi-podophyllotoxin und 10 g (25 mmol) Verbindung 5 wurden in 400 ml Dichlormethan/Essigsäureethylester 1:1 gelöst und mit 23 g Molekularsieb 4 Å versetzt. Zu der auf -18° C abgekühlten Reaktionsmischung wurden unter Rühren 80 ml $BF_3$xEther portionsweise zugegeben. Nach 4 h Rühren bei -18° C wurden weitere 4.0 g Aglycon, gelöst in 50 ml Essigsäureethylester, zugegeben. Nach 10 h wurde der Reaktionsansatz mit 80 ml Triethylamin bei -18° C versetzt. Die Reaktionsmischung wurde abfiltriert und in Vakuum eingedampft. Der Rückstand wurde in Chloroform gelöst und mit Eiswasser ausgewaschen. Die organische Phase wurde über Natriumsulfat getrocknet und in Vakuum eingedampft. Das Produkt wurde säulenchromatographisch über Kieselgel (Eluenz: Dichlormethan/Aceton 15:1) gereinigt. Ausbeute: 17 g (74 %),
$(alpha)_D^{20}$ = + 25° (c=0.2 in $CHCl_3$)
Schmelzpunkt: 140 - 142° C
1H-NMR (300 MHz, H,H-COSY, $CDCl_3$) Delta: 7.30-7.44 (m, 10H, Ph), 6.74 (s, 1H, H-5), 6.50 (s, 1H, H-8), 6.24 (s, 2H, H-2′ und H-6′), 5.93 (s, 1H, H-15a), 5.76 (s, 1H, H-15b), 5.33 (dd, IH, J(2″,3″) = 10 Hz, J(3″,4″) = 10 Hz, H-3″), 5.26 (s, 2H, $Ch_2Ph$), 5.05 (d, 1H, Jg = 12.5 Hz, CH-Ph-A), 4.93 (d, 1H, Jg = 12.5 Hz, CH-Ph-B), 4.89 (d, 1H, J(1″,2″) = 7.5 Hz, H-1″), 4.89 (d, 1H, J(3,4) = 3 Hz, H-4), 4.89 (d, 1H, J(2″,NH) = 9.5 Hz, NH), 4.70 (q, 1H, J(Me,CH) = 5 Hz, Me-CH=), 4.52 (d, 1H, J(1,2) = 5.3 Hz, H-1), 4.45 (dd, 1H, Jg = 9 Hz, J(3,11a) = 10 Hz, H-11a), 4.24 (dd, 1H, Jg, J(3,11b) = 7 Hz, H-11b), 4.22 (dd, 1H, Jg = 10.5 Hz, J-(5″,6″e) = 4.5 Hz, H-6″e), 4.06 (d, 1H, Jg = 15 Hz, ClAc), 3.99 (d, 1H, Jg = 15 Hz, ClAc), 3.66 (s, 3H, MeO), 3.60 (dd, 1H, J(3″,4″) = 9 Hz, J(4″,5″) = 9 Hz, H-5″), 3.23 (dd, 1H, J(2,3) = 14 Hz, J(1,2), H-2), 2.83 (m, 1H, J(2,3), J(3,4), J(3,11a), J(3,11b), H-3), 1.34 (s, 3H, J(Me,CH) = 5 Hz, MeC=).

4-O-(2-N-Benzyloxycarbonyl-3-O-chloracetyl-4,6-O-ethyliden-beta-D-glucosaminyl)-4′-O-chloracetyl-4′-O-demethyl-4-epi-podophyllotoxin (Verbindung 8)

Ausgehend von 200 mg (0.42 mmol) 4′-O-Chloracetyl-4′-O-demethyl-4-epi-podophyllotoxin und 175 mg (0.42 mmol) Verbindung 5 wurde die Titelverbindung nach der Vorschrift zur Herstellung von Verbindung 7 hergestellt und mittels 1H- und 13C-NMR-Spektra charakterisiert. Ausbeute: 257 mg (70 %)

4-O-(2-N-Benzyloxycarbonyl-3-O-chloracetyl-4,6-O-ethyliden-beta-D-glucosaminyl)-4-epi-podophyllotoxin (Verbindung 9)

Ausgehend von 150 mg (0.36 mmol) Podophyllotoxin und 150 mg (0.36 mmol) Verbindung 5 wurde die Titelverbindung nach der Vorschrift zur Herstellung von Verbindung 7 hergestellt und mittels 1H- und 13C-NMR- Spektra charakterisiert. Ausbeute: 190 mg (65 %)

Beispiel 4

Deacylierung der Glucosaminide

4-O-(2-N-Benzyloxycarbonyl-4,6-O-ethyliden-beta-D-glucosaminyl)-4′-O-benzyloxycarbonyl-4′-O-demethyl-4-epi-podophyllotoxin (Verbindung 10)

a) Deacylierung des 3″-O-Chloracetyl-Derivates (Verbindung 7)

2.5 g (2.4 mmol) Verbindung 7 wurden in 500 ml Methanol gelöst und mit 5 g Ionenaustauscher Dowex 1x8 versetzt. Nach 2 h Rühren bei Raumtemperatur wurde abfiltriert und das Harz mit Methanol ausgewaschen. Die organische Phase wurde in Vakuum eingedampft und der in Chloroform gelöste Rückstand wurde ausgewaschen. Die organische Phase wurde über Natriumsulfat getrocknet und in Vakuum eingedampft. Ausbeute: 1.95 g (98 %), $(alpha)_D^{20}$ = -65.4° (c=1 in Chloroform)

Die Titelverbindung wurde NMR-spektroskopisch charakterisiert.

b) Deacylierung des 3″-O-Acetyl-Derivates (Verbindung 6)

600 mg (0.61 mmol) Verbindung 6 wurden in 50 ml trockenem Methanol gelöst und mit 83 mg Zinkdichlorid versetzt. Der Reaktionsansatz wurde 3 d unter Rückfluß gehalten. Nach der üblichen Aufarbeitung wurde das Produkt säulenchromatographisch gereinigt. Ausbeute: 377 mg ( 65 %)

4-O-(2-N-Benzyloxycarbonyl-4,6-O-ethyliden-beta-D-glucosaminyl)-4-epi-podophyllotoxin (Verbindung 11)

190 mg (0.234 mmol) Verbindung 9 wurden mit Dowex nach der Vorschrift zu der Deacylierung von Verbindung 7 zu der Titelverbindung entblockiert. Ausbeute: 160 mg (93 %)

Beispiel 5

Hydrogenolytische Abspaltung der N- bzw. O-Benzyloxycarbonyl-Schutzgruppe

4′-O-Demethyl-4-epi-4-O-(4,6-O-ethyliden-beta-D-glucosaminyl)-podophyllotoxin (Verbindung 12)

1.5 g (1.88 mmol) Verbindung 10 wurden in 100 ml Methanol gelöst und mit 0.45 g Palladium/Kohle versetzt. Der Reaktionsansatz wurde 1 h bei Raumtemperatur unter Normaldruck hydriert. Der Ansatz wurde abfiltriert und im Vakuum eingedampft. Das Produkt kristallisiert aus Methanol/Essigsäureethylester aus. Ausbeute: 0.95 g (90 %), $(alpha)_D^{20}$ = -115.4° (c=1 in Methanol)
Schmelzpunkt: 202 - 204° C

4-Epi-4-O-(4,6-O-ethyliden-beta-D-glucosaminyl)-podophyllotoxin (Verbindung 13)

Ausgehend von 160 mg (0.217 mmol) Verbindung 11 wurde die Titelverbindung nach der Vorschrift zur Herstellung von Verbindung 12 hydriert und aufgearbeitet. Ausbeute: 117 mg (90 %)

Beispiel 6

Reduktive Alkylierung der 2″-Amino-Derivate

4′-O-Demethyl-4-O-(2-N,N-dimethyl-4,6-O-ethyliden-beta-D-glucosaminyl)-4-epi-podophyllotoxin (Verbindung 14)

2.5 g (4.25 mmol) Verbindung 12 wurden in 200 ml Methanol gelöst und mit 4 ml einer 37%igen wäßrigen Formaldehyd-Lösung und 1.06 g Natriumcyanoborhydrid versetzt. Nach 1 h Rühren wurde der Reaktionsansatz in Vakuum eingedampft und der Rückstand wurde säulenchromatographisch über Kieselgel (Eluenz: Dichlormethan/Methanol 10:1) gereinigt. Ausbeute: 2 g (76 %), $(alpha)_D^{20}$ = -113.2° (c=1 in Chloroform)

4-O-(2-N,N-Dimethyl-4.6-O-ethyliden-beta-D-glucosaminyl)4-epi-podophyllotoxin (Verbindung 15)

Ausgehend von 110 mg (0.18 mmol) Verbindung 13 wurde die Titelverbindung nach der Vorschrift zur Herstellung von Verbindung 14 hergestellt. Ausbeute: 95 mg (75 %)

## Ansprüche

1. Verfahren zur Herstellung eines Glucosaminyl-epi-podophyllotoxin-Derivates der Formel I

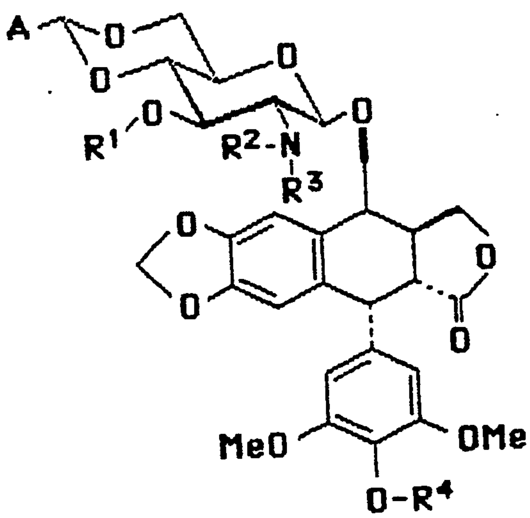

I

worin
$R^1$ Wasserstoff oder die Acetyl- oder eine Mono-, Di- oder Trihalogenacetyl- Schutzgruppe mit Halogen Fluor, Chlor oder Brom,
$R^2$ Wasserstoff oder ($C_1$-$C_4$)-Alkyl,
$R^3$ Wasserstoff, ($C_1$-$C_4$)-Alkyl oder die Benzyloxycarbonyl-Schutzgruppe,
$R^4$ Wasserstoff, eine Mono-, Di- oder Trihalogenacetyl-Schutzgruppe, eine Benzyloxycarbonyl-Schutzgruppe oder eine Methylgruppe und
A $C_1$-$C_4$-Alkyl
bedeuten, dadurch gekennzeichnet, daß man ein in der alpha-Hydroxy-Form vorliegende Glucosamin-Derivat der Formel II

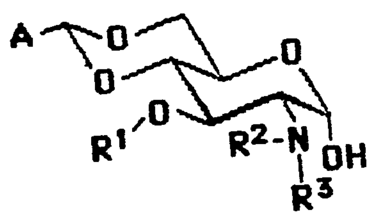

II

worin
$R^1$ und $R^3$ wie oben definiert Acyl-Schutzgruppen sind und
$R^2$ Wasserstoff und
A $C_1$-$C_4$-Alkyl bedeuten
mit einem Podophyllotoxin-Derivat der Formel III

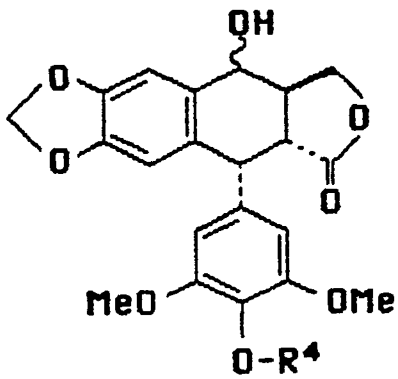

III

worin
$R^4$ eine Methylgruppe oder eine Mono-, Di- oder Trihalogenacetyl- oder Benzyloxycarbonyl-Schutzgruppe darstellt,
in der Gegenwart eines Promotors wie $BF_3$xEther oder ein Tri-($C_1$-$C_4$)-alkyl-silyltrifluormethansulfonates und eines wasserfreien organischen Lösungsmittels bei -50° C bis 20° C zu einem 4-O-(beta-Glucosaminyl)-epi-Podophyllotoxin-Derivat der Formel I,
in der die Reste $R^1$, $R^2$, $R^3$, $R^4$ und A ihre Bedeutung, wie oben definiert, beibehalten, umsetzt, anschließend die Benzyloxycarbonyl-Schutzgruppe hydrogenolytisch mittels eines Palladium-Katalysators, die Acetylschutzgruppe mittels Zink-dichlorid oder -diacetat und die Halogenacetylschutzgruppen mittels eines basischen Anionenaustauschers in einem organischen Lösungsmittel, wie Methanol, Ethanol, Aceton, Essigsäureethylester oder deren gegebenenfalls wäßrigen Mischungen abspaltet und gegebenenfalls die freie Aminogruppe unter den Bedingungen der reduktiven Alkylierung mit einem $C_1$-$C_4$-Aldehyd in der Gegenwart eines Hydrides zu einem Mono- oder Dialkylamino-Derivat umsetzt, wobei ein 4-O-(beta-Glucosaminyl)-epi-podophyllotoxin-Derivat der Formel I, worin
$R^1$ Wasserstoff,
$R^2$ und $R^3$ Wasserstoff oder ($C_1$-$C_4$)-Alkyl,
$R^4$ Wasserstoff oder die Methylgruppe und
A $C_1$-$C_4$-Alkyl
darstellen, gebildet werden.

2. Verfahren nach Anspruch 1, worin
$R^1$ Wasserstoff oder die Acetyl- oder eine Chloracetyl-Schutzgruppe,
$R^2$ Wasserstoff oder ($C_1$-$C_4$)-Alkyl,
$R^3$ Wasserstoff, ($C_1$-$C_4$)-Alkyl oder die Benzyloxycarbonyl-Schutzgruppe,
$R^4$ Wasserstoff, Methyl oder Benzyloxycarbonyl- oder Chloracetyl- Schutzgruppen und
A Methyl bedeuten,
dadurch gekennzeichnet, daß man ein in der alpha-Hydroxy-Form vorliegendes Glucosamin-Derivat der Formel II, worin
$R^1$ eine Chloracetylgruppe,
$R^2$ ein Wasserstoffatom,
$R^3$ eine Benzyloxycarbonylgruppe und
A eine Methylgruppe
darstellt, mit einem Podophyllotoxin-Derivat der Formel III, worin
$R^4$ eine Methylgruppe, Chloracetyl- oder Benzyloxycarbonylschutzgruppe
ist, in der Gegenwart von $BF_3$xEther oder Tri-($C_1$-$C_4$)-alkyl-silyltrifluormethansulfonates in einem organischen Lösungsmittel bei -30°C bis -10°C zu einem 4-O-(beta-Glucosaminyl)-epi-Podophyllotoxin-Derivat der Formel I umsetzt und die Verbindung zur Herstellung einer weiteren Verbindung der Formel I verwendet.

3. Verfahren nach mindestens einem der Ansprüche 1 und 2, worin
$R^1$ die Acetyl- oder eine Mono-, Di- oder Trihalogenacetyl-Schutzgruppe mit Halogen Fluor, Chlor oder Brom,
$R^2$ ein Wasserstoffatom,
$R^3$ eine Benzyloxycarbonyl-Schutzgruppe,
$R^4$ eine Mono-, Di- oder Trihalogenacetyl- oder Benzyloxycarbonyl-Schutzgruppe oder Methylgruppe und
A $C_1$-$C_4$-Alkyl

darstellen, dadurch gekennzeichnet, daß man ein in der alpha-Form vorliegendes Glucosamin-Derivat der Formel II, worin $R^1$, $R^2$, $R^3$ und A, wie oben definiert sind, mit einem Podophyllotoxin-Derivat der Formel III, worin $R^4$, wie oben definiert ist, in der Gegenwart eines Promotors wie $BF_3$xEther oder ein Tri-($C_1$-$C_4$)-alkyl-silyltrifluormethansulfonat und eines wasserfreien organischen Lösungsmittels bei -50° C bis +20° C, bevorzugt bei -20° C bis -30° C, gegebenenfalls unter Zugabe eines Trockenmittels wie eines Molekularsieb umsetzt.

4. Verfahren nach Anspruch 3, worin als Promotor $BF_3$xEther eingesetzt wird.

5. Verfahren nach Anspruch 3, worin als organisches Lösungsmittel Essigsäureethylester oder dessen Mischung mit einem anderen Lösungsmittel wie Dichlormethan, Aceton oder Ether verwendet wird.

6. Verfahren zur Herstellung eines in der alpha-Form vorliegenden Glucosamin-Derivates der Formel II, worin
$R^1$ eine Acyl-Schutzgruppe
$R^2$ Wasserstoff
$R^3$ Benzyloxycarbonyl-Schutzgruppe und
A $C_1$-$C_4$-Alkyl
darstellen, dadurch gekennzeichnet, daß man ein 1,3-Di-O-acyl-4,6-O-alkyliden-2-N-benzyloxycarbonyl-alpha,beta-D-glucosamin-Derivat der Formel IV

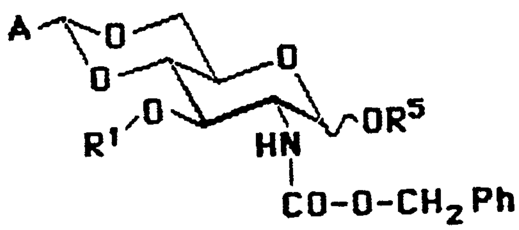

IV

worin
$R^1$ und $R^5$ eine wie oben definierte Acyl-Schutzgruppe,
$R^2$ Wasserstoff,
$R^3$ die Benzyloxycarbonyl-Schutzgruppe und
A $C_1$-$C_4$-Alkyl
darstellen, mit einer organischen Base wie Piperidin oder Pyridin oder mit Kieselgel in einem polaren Lösungsmittel wie Methanol oder Ethanol behandelt, wobei eine Verbindung der Formel II entsteht.

7. 3-O-Acyl-4,6-O-alkyliden-2-N-benzyloxycarbonyl-alpha-D-glucosamin-Derivat der Formel II, worin
$R^1$ Acetyl- oder Mono-, Di- oder Trihalogenacetyl mit Halogen Fluor, Chlor oder Brom und
A $C_1$-$C_4$-Alkyl darstellen.

8. Derivat nach Anspruch 7, worin
$R^1$ die Acetyl- oder Chloracetyl-Schutzgruppe und
A Methyl darstellen.

9. 4-O-(4,6-O-Alkyliden-beta-D-glucosaminyl)-4-epi-podophyllotoxin der Formel I in Anspruch 1 mit A $C_1$-$C_4$-Alkyl.

10. 4-O-(4,6-O-Alkyliden-2-N-dimethyl-beta-D-glucosaminyl)-4-epi-podophyllotoxin der Formel I in Anspruch 1 mit A $C_1$-$C_4$-Alkyl.

Patentansprüche für folgende Vertragsstaaten : ES, GR

1. Verfahren zur Herstellung eines Glucosaminyl-epi-podophyllotoxin-Derivates der Formel I

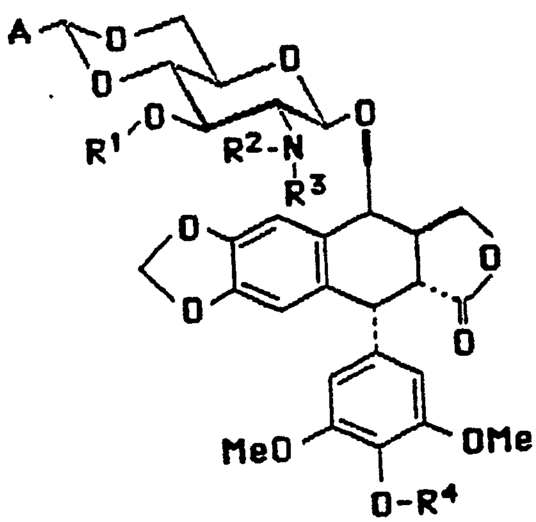

I

worin
$R^1$ Wasserstoff oder die Acetyl- oder eine Mono-, Di- oder Trihalogenacetyl- Schutzgruppe mit Halogen Fluor, Chlor oder Brom,
$R^2$ Wasserstoff oder $(C_1-C_4)$-Alkyl,
$R^3$ Wasserstoff, $(C_1-C_4)$-Alkyl oder die Benzyloxycarbonyl-Schutzgruppe,
$R^4$ Wasserstoff, eine Mono-, Di- oder Trihalogenacetyl-Schutzgruppe, eine Benzyloxycarbonyl-Schutzgruppe oder eine Methylgruppe und
A $C_1-C_4$-Alkyl
bedeuten, dadurch gekennzeichnet, daß man ein in der alpha-Hydroxy-Form vorliegende Glucosamin-Derivat der Formel II

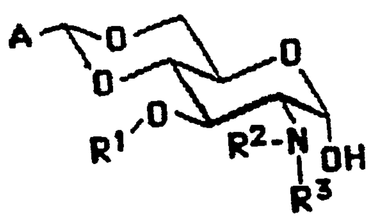

II

worin
$R^1$ und $R^3$ wie oben definiert Acyl-Schutzgruppen sind und
$R^2$ Wasserstoff und
A $C_1-C_4$-Alkyl bedeuten
mit einem Podophyllotoxin-Derivat der Formel III

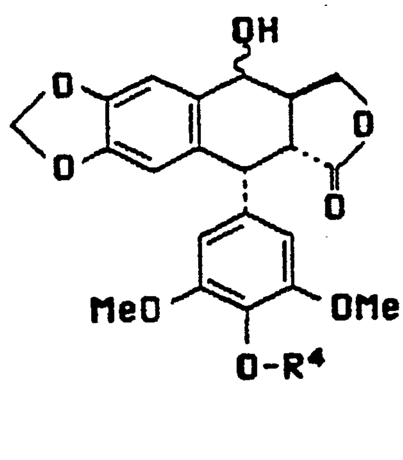

III

worin
$R^4$ eine Methylgruppe oder eine Mono-, Di- oder Trihalogenacetyl- oder Benzyloxycarbonyl-Schutzgruppe

darstellt,
in der Gegenwart eines Promotors wie $BF_3$xEther oder ein Tri-($C_1$-$C_4$)-alkyl-silyltrifluormethansulfonates und eines wasserfreien organischen Lösungsmittels bei -50° C bis 20° C zu einem 4-O-(beta-Glucosaminyl)-epi-Podophyllotoxin-Derivat der Formel I,
in der die Reste $R^1$, $R^2$, $R^3$, $R^4$ und A ihre Bedeutung, wie oben definiert, beibehalten, umsetzt, anschließend die Benzyloxycarbonyl-Schutzgruppe hydrogenolytisch mittels eines Palladium-Katalysators, die Acetylschutzgruppe mittels Zink-dichlorid oder -diacetat und die Halogenacetylschutzgruppen mittels eines basischen Anionenaustauschers in einem organischen Lösungsmittel, wie Methanol, ethanol, Aceton, Essigsäureethylester oder deren gegebenenfalls wäßrigen Mischungen abspaltet und gegebenenfalls die freie Aminogruppe unter den Bedingungen der reduktiven Alkylierung mit einem $C_1$-$C_4$-Aldehyd in der Gegenwart eines Hydrides zu einem Mono- oder Dialkylamino-Derivat umsetzt, wobei ein 4-O-(beta-Glucosaminyl)-epi-podophyllotoxin-Derivat der Formel I, worin
$R^1$ Wasserstoff,
$R^2$ und $R^3$ Wasserstoff oder ($C_1$-$C_4$)-Alkyl,
$R^4$ Wasserstoff oder die Methylgruppe und
A $C_1$-$C_4$-Alkyl
darstellen, gebildet werden.

2. Verfahren nach Anspruch 1, worin
$R^1$ Wasserstoff oder die Acetyl- oder eine Chloracetyl-Schutzgruppe,
$R^2$ Wasserstoff oder ($C_1$-$C_4$)-Alkyl,
$R^3$ Wasserstoff, ($C_1$-$C_4$)-Alkyl oder die Benzyloxycarbonyl-Schutzgruppe,
$R^4$ Wasserstoff, Methyl oder Benzyloxycarbonyl- oder Chloracetyl- Schutzgruppen und
A Methyl bedeuten,
dadurch gekennzeichnet, daß man ein in der alpha-Hydroxy-Form vorliegendes Glucosamin-Derivat der Formel II, worin
$R^1$ eine Chloracetylgruppe,
$R^2$ ein Wasserstoffatom,
$R^3$ eine Benzyloxycarbonylgruppe und
A eine Methylgruppe
darstellt, mit einem Podophyllotoxin-Derivat der Formel III, worin
$R^4$ eine Methylgruppe, Chloracetyl- oder Benzyloxycarbonylschutzgruppe
ist, in der Gegenwart von $BF_3$xEther oder Tri-($C_1$-$C_4$)-alkyl-silyltrifluormethansulfonates in einem organischen Lösungsmittel bei -30° C bis -10° C zu einem 4-O-(beta-Glucosaminyl)-epi-Podophyllotoxin-Derivat der Formel I umsetzt und die Verbindung zur Herstellung einer weiteren Verbindung der Formel I verwendet.

3. Verfahren nach mindestens einem der Ansprüche 1 und 2, worin
$R^1$ die Acetyl- oder eine Mono-, Di- oder Trihalogenacetyl-Schutzgruppe mit Halogen Fluor, Chlor oder Brom,
$R^2$ ein Wasserstoffatom,
$R^3$ eine Benzyloxycarbonyl-Schutzgruppe,
$R^4$ eine Mono-, Di- oder Trihalogenacetyl- oder Benzyloxycarbonyl-Schutzgruppe oder Methylgruppe und
A $C_1$-$C_4$-Alkyl
darstellen, dadurch gekennzeichnet, daß man ein in der alpha-Form vorliegendes Glucosamin-Derivat der Formel II, worin $R^1$, $R^2$, $R^3$ und A, wie oben definiert sind, mit einem Podophyllotoxin-Derivat der Formel III, worin $R^4$, wie oben definiert ist, in der Gegenwart eines Promotors wie $BF_3$xEther oder ein Tri-($C_1$-$C_4$)-alkyl-silyltrifluormethansulfonat und eines wasserfreien organischen Lösungsmittels bei -50° C bis +20° C, bevorzugt bei -20° C bis -30° C, gegebenenfalls unter Zugabe eines Trockenmittels wie eines Molekularsieb umsetzt.

4. Verfahren nach Anspruch 3, worin als Promotor $BF_3$xEther eingesetzt wird.

5. Verfahren nach Anspruch 3, worin als organisches Lösungsmittel Essigsäureethylester oder dessen Mischung mit einem anderen Lösungsmittel wie Dichlormethan, Aceton der Ether verwendet wird.

6. Verfahren zur Herstellung eines in der alpha-Form vorliegenden Glucosamin-Derivates der Formel II, worin
$R^1$ eine Acyl-Schutzgruppe
$R^2$ Wasserstoff
$R^3$ Benzyloxycarbonyl-Schutzgruppe und
A $C_1$-$C_4$-Alkyl
darstellen, dadurch gekennzeichnet, daß man ein 1,3-Di-O-acyl-4,6-O-alkyliden-2-N-benzyloxycarbonyl-alpha,beta-D-glucosamin-Derivat der Formel IV

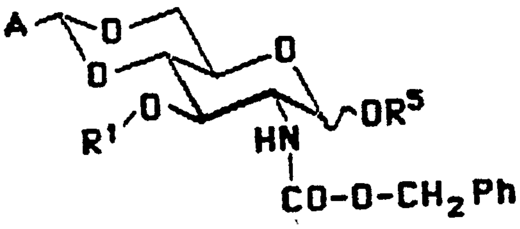

IV

worin
$R^1$ und $R^5$ eine wie oben definierte Acyl-Schutzgruppe,
$R^2$ Wasserstoff,
$R^3$ die Benzyloxycarbonyl-Schutzgruppe und
A $C_1$-$C_4$-Alkyl
darstellen, mit einer organischen Base wie Piperidin oder Pyridin oder mit Kieselgel in einem polaren Lösungsmittel wie Methanol oder Ethanol behandelt, wobei eine Verbindung der Formel II entsteht.

Europäisches Patentamt

# EUROPÄISCHER RECHERCHENBERICHT

Nummer der Anmeldung

EP 90107638.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | CHEMISTRY LETTERS, Nr. 1, 1987<br>H. SAITO et al. "Synthesis of all four possible diastereomers of etoposide and its aminoglycosidic analogues have been achieved via optical resolution of (+)-podophyllotoxin by glycosidation with D- and L-sugars."<br>Seiten 799-802<br>* Gesamt *<br>-- | 1-10 | C 07 H 17/04<br>C 07 H 15/12<br>//A 61 K 31/70 |
| D,A | EP - A1 - 0 141 057<br>(MICROBIAL CHEMISTRY RESEARCH FOUND.)<br>* Ansprüche *<br>-- | 1-5,9,10 | |
| D,A | EP - A1 - 0 196 618<br>(MICROBIAL CHEMISTRY RESEARCH FOUND.)<br>* Ansprüche *<br>---- | 1-5,9,10 | RECHERCHIERTE SACHGEBIETE (Int Cl 5)<br>C 07 H 17/00<br>C 07 H 15/00 |

Der vorliegende Recherchenbericht wurde für alle Patentansprüche erstellt.

| Recherchenort | Abschlußdatum der Recherche | Prüfer |
|---|---|---|
| WIEN | 26-07-1990 | JANISCH |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82